# EUROPEAN PATENT SPECIFICATION

(11) **EP 3 239 708 B1**
(45) Date of publication and mention of the grant of the patent: **06.01.2021**
(21) Application number: 17169362.5
(22) Date of filing: 26.05.2011
(51) Int. Cl.: G01N 33/50

(54) **SINGLE B-CELL CULTIVATION METHOD**
VERFAHREN ZUR KULTIVIERUNG VON EINZEL-B-ZELLEN
PROCÉDÉ DE CULTURE DE LYMPHOCYTE SIMPLE

(30) Priority: 28.05.2010 EP 10005602
(43) Date of publication of application: 01.11.2017
(62) Divisional of application: 11722402.2
(73) Proprietor: F. Hoffmann-La Roche AG, 4070 Basel (CH)
(72) Inventor: ENDL, Josef, 82362 Weilheim (DE); SCHUHMACHER, Natalie, 68794 Oberhausen/Rheinhausen (DE); OFFNER, Sonja, 82377 Penzberg (DE); PLATZER, Josef, 82538 Geretsried (DE); SIEWE, Basile, Des Plaines, IL 60018 (US); THOREY, Irmgard, 82362 Weilheim (DE)
(74) Representative: Burger, Alexander

(56) References cited:
- PATEL K D ET AL: "High sensitivity cytokine detection in acute coronary syndrome reveals up-regulation of Interferon Gamma and Interleukin-10 post Myocardial Infarction", CLINICAL IMMUNOLOGY, ACADEMIC PRESS, US, vol. 133, no. 2, 1 November 2009 (2009-11-01), pages 251-256, XP026676990, ISSN: 1521-6616, DOI: 10.1016/J.CLIM.2009.07.007 [retrieved on 2009-08-08]
- FABRICE BOVIA ET AL: "Efficient transduction of primary human B lymphocytes and nondividing myeloma B cells with HIV-1-derived lentiviral vectors", BLOOD, AMERICAN SOCIETY OF HEMATOLOGY, US, vol. 101, no. 5, 1 January 2003 (2003-01-01), pages 1727-1733, XP008155464, ISSN: 0006-4971, DOI: 10.1182/BLOOD-2001-12-0249 [retrieved on 2002-10-24]
- A Tucci ET AL: "Effects of eleven cytokines and of IL-1 and tumor necrosis factor inhibitors in a human B cell assay", The Journal of Immunology, 1 May 1992 (1992-05-01), pages 2778-2784, XP055375747, UNITED STATES Retrieved from the Internet: URL:http://www.jimmunol.org/content/148/9/ 2778.full-text.pdf [retrieved on 2017-09-01]

## Description

Herein is reported a method for obtaining the amino acid sequence of at least the variable domains of a monoclonal antibody secreted by a single B-cell that has been obtained from a population of B-cells from an experimental animal by single cell deposition and co-cultivation with feeder cells in the presence of a feeder mix.

### Background of the Invention

For obtaining cells secreting monoclonal antibodies the hybridoma technology developed by Koehler and Milstein is widely used. But in the hybridoma technology only a fraction of the B-cells obtained from an immunized experimental animal can be fused and propagated. The source of the B-cells is generally an organ of an immunized experimental animal such as the spleen.

Zubler et al. started in 1984 to develop a different approach for obtaining cells secreting monoclonal antibodies (see e.g. Eur. J. Immunol. 14 (1984) 357-63, J. Exp. Med. 160 (1984) 1170-1183). Therein the B-cells are obtained from the blood of the immunized experimental animal and co-cultivated with murine EL-4 B5 feeder cells in the presence of a cytokine comprising feeder mix. With this methodology up to 50 ng/ml antibody can be obtained after 10-12 days of co-cultivation.

Weitkamp, J-H., et al., (J. Immunol. Meth. 275 (2003) 223-237) report the generation of recombinant human monoclonal antibodies to rotavirus from single antigen-specific B-cells selected with fluorescent virus-like particles. A method of producing a plurality of isolated antibodies to a plurality of cognate antigens is reported in US 2006/0051348. In WO 2008/144763 and WO 2008/045140 antibodies to IL-6 and uses thereof and a culture method for obtaining a clonal population of antigen-specific B cells are reported, respectively. A culture method for obtaining a clonal population of antigen-specific B-cells is reported in US 2007/0269868. Masri et al. (in Mol. Immunol. 44 (2007) 2101-2106) report the cloning and expression in E.coli of a functional Fab fragment obtained from single human lymphocyte against anthrax toxin. A method for preparing immunoglobulin libraries is reported in WO 2007/ 031550.

Patel, K.D., et al. (Clin. Immunol. 133 (2009) 251-256) reported high sensitivity cytokine detection in acute coronary syndrome reveals up-regulation of interferon gamma and interleukin-10 post myocardial infarction.

Bovia, F., et al. reported the efficient transduction of primary human B lymphocytes and non-dividing myeloma B cells with HIV-1-derived lentiviral vectors (Blood 101 (2003) 1727-1733).

A feeder mix consisting of IL-1β (interleukin-1 beta), TNFα (tumor necrosis factor alpha), IL-2 (interleukin-2) and IL-10 (interleukin-10) is known from Tucci, A., et al., J. Immunol. 148 (1992) 2778-2784.

### Summary of the Invention

The invention is defined by the claims.

Herein is reported a method for the isolation of a B-cell from a population of B-cells that has special properties. First already within four weeks after the first immunization of an experimental animal the induced antibody producing cells can be isolated and the binding specificity of the antibodies can be determined. Second it is possible to enhance the number and/or the quality (e.g. the antibody production/secretion capacity) of antibody producing cells by any one of the following steps: i) a pre-incubation step, and/or ii) a centrifugation step, and/or iii) a panning step. Third, the feeder mix used for the co-cultivation of B-cells and feeder cells can be improved by the addition of IL-21, or IL-6, or SAC, or BAFF.

Subject matter of the current invention is the use of a synthetic feeder mix for the co-cultivation of B-cells and feeder cells, wherein the synthetic feeder mix is consisting of synthetic components comprising IL-1β, TNFα, IL-10, and
- IL-2 and SAC, or
- IL-6, wherein the feeder mix is for the co-cultivation of rabbit B-cells and feeder cells, or
- IL-6 or IL-2, and SAC, wherein the feeder mix is for the co-cultivation of rabbit B-cells and feeder cells, or
- IL-2 and IL-6, wherein the feeder mix is for the co-cultivation of murine or hamster B-cells and feeder cells, or
- IL-2, IL-6 and SAC, wherein the feeder mix is for the co-cultivation of hamster B-cells and feeder cells.

In one embodiment of all aspects as reported herein the use comprises the step of incubating the population of B-cells in the co-cultivation medium prior to single cell depositing. In one embodiment the incubating is at about 37 °C. In one embodiment the incubating is for 0.5 to two hours. In a specific embodiment the incubating is for about one hour. In one embodiment the incubating is at about 37 °C for about one hour.

In one embodiment of all aspects as reported herein the use comprises the step of centrifuging the single cell deposited B-cells prior to the co-cultivation. In one embodiment the centrifuging is for about 1 min. to about 30 min. In a specific embodiment the centrifuging is for about 5 min. In one embodiment the centrifuging is at about 100 x g to about 1,000 x g. In a specific embodiment the centrifuging is at about 300 x g. In one embodiment the centrifuging is for about 5 min. at about 300 x g.

In one embodiment of all aspects as reported herein the use comprises immediately prior to the labeling step the following step: panning the B-cells with immobilized antigen.

In one embodiment of all aspects as reported herein the population of B-cells is obtained from the blood of an animal by a density gradient centrifugation.

In one embodiment of all aspects as reported herein the population of B-cells is obtained from the blood of an experimental animal after 4 days after the immunization. In another embodiment the population of B-cells is obtained from the blood of an experimental animal of from 4 days to at least 9 days after immunization. In a further embodiment the population of B-cells is obtained from the blood of an experimental animal of from 4 days to 9 days after immunization.

In one embodiment of all aspects as reported herein the population of B-cells is isolated by density gradient centrifugation.

In one embodiment of all aspects as reported herein the B-cells are mature B-cells.

In one embodiment of all aspects as reported herein the labeling is with one to three fluorescence dyes. In a specific embodiment the labeling is with two or three fluorescence dyes.

In one embodiment of all aspects as reported herein the labeling of the B-cells results in labeling of 0.1 % to 2.5 % of the cells of the total B-cell population.

In one embodiment of all aspects as reported herein the B-cells are mouse B-cells, or hamster B-cells, or rabbit B-cells.

In one embodiment of all aspects as reported herein the single cell depositing is in the wells of a multi well plate.

In one embodiment of all aspects as reported herein the feeder cells are murine EL-4 B5 cells.

In one embodiment of all aspects as reported herein the antibody is a monoclonal antibody.

In one embodiment of all aspects as reported herein the labeling is of IgG⁺CD19⁺-B-cells, IgG⁺CD38⁺-B-cells, IgG⁺CD268⁺-B-cells, IgG⁻CD138⁺-B-cells, CD27⁺CD138⁺-B-cells, or CD3⁻CD27⁺-B-cells.

In one embodiment of all aspects as reported herein the B-cells are of mouse origin and the labeling is of IgG⁺CD19⁺-B-cells, and/or IgG⁻CD138⁺-B-cells.

In one embodiment of all aspects as reported herein the B-cells are of hamster origin and the labeling is of IgG⁺IgM⁻-B-cells.

In one embodiment of all aspects as reported herein the B-cells are of rabbit origin and the labeling is of IgG⁺-B-cells and/or CD138⁺-B-cells, or CD138⁺IgG⁺-B-cells and/or IgG⁺IgM⁻-B-cells.

In one embodiment of all aspects as reported herein the co-cultivating is in an RPMI 1640 medium supplemented with 10 % (v/v) FCS, 1 % (w/v) of a 200 mM glutamine solution that comprises penicillin and streptomycin, 2 % (v/v) of a 100 mM sodium pyruvate solution, and 1 % (v/v) of a 1 M 2-(4-(2-hydroxyethyl)-1-piperazine)-ethane sulfonic acid (HEPES) buffer. In another embodiment the co-cultivating medium further comprises 0.05 mM beta-mercaptoethanol.

The co-cultivating of the B-cells is with feeder cells and a synthetic feeder mix.

The synthetic feeder mix comprises interleukin-1 beta and tumor necrosis factor alpha. In one embodiment the synthetic feeder mix comprises interleukin-2 (IL-2) and interleukin-10 (IL-10). In one embodiment the synthetic feeder mix further comprises Staphylococcus aureus strain Cowans cells (SAC). In one embodiment the synthetic feeder mix comprises interleukin-6 (IL-6). In one embodiment the synthetic feeder mix comprises interleukin-4 (IL-4).

In one embodiment the experimental animal is selected from mouse, hamster, and rabbit.

### Detailed Description of the Invention

The use according to the invention allows for a rapid characterization of the binding specificity of monoclonal antibodies obtained from individual B-cell clones, i.e. within four weeks after the first immunization of the experimental animal the induced antibody producing cells can be isolated and the binding specificity of the antibodies produced therefrom can be determined, whereby at least 4 different experiments can be performed due to the antibody amount/concentration in the B-cell co-cultivation supernatant.

### Immunization:

Often non-human animals, such as mice, rabbits, hamster and rats, are used as animal model for evaluating antibody based therapies. Therefore, it is often required to provide cross-reactive antibodies binding to the non-human animal antigen as well as to the human antigen. The use according to the invention can be used to provide cross-reactive antibodies. In the use according to the invention B-cells obtained from e.g. mouse, hamster and rabbit can be used. In one embodiment the mouse is an NMRI-mouse or a balb/c-mouse. In another embodiment the hamster is selected from Armenian hamster (Cricetulus migratorius), Chinese hamster (Cricetulus griseus), and Syrian hamster (Mesocricetulus auratus). In a specific embodiment the hamster is the Armenia hamster. In one embodiment the rabbit is selected from New Zealand White (NZW) rabbits, Zimmermann-rabbits (ZIKA), Alicia-mutant strain rabbits, basilea mutant strain rabbits, transgenic rabbits with a human immunoglobulin locus, rbIgM knock-out rabbits, and cross-breeding thereof.

In one embodiment the experimental animals, e.g. mice, hamster and rabbits, chosen for immunization are not older than 12 weeks.

### Source and isolation of B-cells:

The blood of an experimental animal provides a high diversity of antibody producing B-cells. The therefrom obtained B-cells secrete antibodies that have almost no identical or overlapping amino acid sequences within the CDRs, thus, show a high diversity.

In one embodiment the B-cells of an experimental animal, e.g. from the blood, are obtained of from 4 days after immunization until at least 9 days after immunization or the most recent boost. This time span allows for a high flexibility in the use according to the invention. In this time span it is likely that the B-cells providing for the most affine antibodies migrate from spleen to blood (see e.g. Paus, D., et al., JEM 203 (2006) 1081-1091; Smith, K.G.S., et al., The EMBO J. 16 (1997) 2996-3006; Wrammert, J., et al., Nature 453 (2008) 667-672).

B-cells from the blood of an experimental animal may be obtained with any method known to a person skilled in the art. For example, density gradient centrifugation (DGC) or red blood cell lysis (lysis) can be used. Density gradient centrifugation compared to hypotonic lysis provides for a higher overall yield, i.e. number of B-cell clones. Additionally from the cells obtained by density gradient centrifugation a larger number of cells divides and grows in the co-cultivation step. Also the concentration of secreted antibody is higher compared to cells obtained with a different method. Therefore, in one embodiment the providing of a population of B-cells is by density gradient centrifugation.

**Table 1: Number of IgG producing wells/cell clones when the cells are obtained by density gradient centrifugation (DGC) or hypotonic lysis of erythrocytes.**

| | **mouse, DGC** | **mouse, lysis** | **hamster, DGC** | **hamster, lysis** |
|---|---|---|---|---|
| number of isolated cells [x 10⁶] | 1.7 ± 0.2 (n= 2) | 1.6 ± 0.1 (n= 2) | 2.1 ± 0.2 (n= 2) | 0.9 ± 0.1 (n= 2) |
| IgG⁺-wells [%] | 22 | 12 | 7 | 6 |

### Selection steps prior to co-cultivation:

B-cells producing antibodies that specifically bind an antigen can be enriched from peripheral blood mononuclear cells (PBMCs). Thus, in one embodiment of all uses the B-cell population is enriched from peripheral blood mononuclear cells (PBMCs).

The term "specifically binding" and grammatical equivalents thereof denote that the antibody binds to its target with a dissociation constant (Kd) of 10⁻⁷ M or less, of from 10⁻⁸ M to 10⁻¹³ M, of from 10⁻⁹ M to 10⁻¹³ M. The term is further used to indicate that the antibody does not specifically bind to other biomolecules present, i.e. it binds to other biomolecules with a dissociation constant (Kd) of 10⁻⁶ M or more, of from 10⁻⁶ M to 1 M.

In one embodiment of the use the PBMCs are depleted of macrophages. This is advantageous as outlined below, e.g. as in one embodiment for B-cells of rabbit origin, for the co-cultivation step.

Macrophages can be depleted from PBMCs by adhesion to the surface of the cell culture plate (see preincubation step).

In one embodiment of the use the cells are from a protein-immunized animal and are depleted of macrophages prior to the labeling.

It has been found that incubating the population of B-cells in co-cultivation medium prior to the single cell depositing increases the total number of antibody secreting cells obtained after the single cell depositing compared to a single cell depositing directly after the isolation and optional enrichment of the population of B-cells from the blood of an experimental animal (example rabbit, see Tables 2a and 2b). Specifically the incubating is at about 37 °C for about one hour in EL-4 B5 medium, e.g. using a cell culture incubator.

**Table 2a: IgG positive wells/cell clones with and without one hour incubation in EL-4 B5 medium prior to single cell depositing of all cells (rb=rabbit).**

| **rbIgG ELISA** | **fresh PBMCs (Ø 100-20 cells)** | **PBLs after incubation* (Ø 50-10 cells)** |
|---|---|---|
| rbIgG⁺ wells [n] | 40 | 108 |
| rbIgG⁺ wells [% total wells] | 28 | 75 |

| | | |
|---|---|---|
| * depleted of macrophages and monocytes | | |

**Table 2b: IgG positive wells/cell clones with and without one hour incubation in EL-4 B5 medium prior to single cell depositing of B-cells.**

| **rbIgG ELISA** | **single B-cells from fresh PBMCs** | **single B-cells from blood, 1 h incubated** | **single B-cells from fresh spleen cells** | **single B-cells from spleen, 1 h incubated** |
|---|---|---|---|---|
| rbIgG⁺ wells [n] | 2 | 55 | 6 | 52 |
| rbIgG⁺ wells [% of total wells] | 2 | 33 | 7 | 31 |

In one embodiment of the use the cells are obtained from a protein-immunized animal and depleted of macrophages.

Cells not producing an antibody binding the antigen or, likewise, cells producing an antibody binding to the antigen can be reduced or enriched, respectively, by using a panning approach. Therein a binding partner is presented attached to a surface and cells binding thereto are selectively enriched in the cell population in case the bound cells are processed further, or reduced in the cell population in case the cells remaining in solution are processed further.

**Table 3: Enrichment of B-cells secreting an antigen-specific antibody by panning with the respective antigen.**

| **protein antigen** | **without panning** | **with panning using the antigen** |
|---|---|---|
| total wells [n] | 4284 | 2113 |
| antigen specific IgG⁺ wells [n] | 235 | 419 |
| antigen specific IgG⁺ wells [% total wells] | 5 | 20 |

| **small molecule antigen** | **without panning** | **with panning using the small molecule** |
|---|---|---|
| total wells [n] | 336 | 336 |
| small molecule IgG⁺ wells [n] | 2 | 115 |
| small molecule IgG⁺ wells [% total wells] | 1 | 34 |

The use comprises in one embodiment prior to the single cell depositing a selecting step in which B-cells producing specific and/or non-cross-reactive antibodies are selected based on cell surface markers and fluorescence activated cell sorting/gating. In one embodiment mature B-cells are sorted/enriched/selected. For selection of B-cells from different experimental animal species different cell surface markers can be used. It has been found that many of the available cell surface markers, either individually or in combination, do not provide for a suitable labeling.

With the labeling of non-target cell populations and non-specifically binding lymphocytes it is possible to selectively deplete these cells. In this depletion step only a non total depletion can be achieved. Albeit the depletion is not quantitative it provides for an advantage in the succeeding fluorescence labeling of the remaining cells as the number of interfering cells can be reduced or even minimized. By a single cell depositing of mature B-cells (memory B-cells, affinity matured plasmablasts and plasma cells) by fluorescence activated cell sorting using the labeling as outlined below a higher number of IgG⁺-wells/cell clones can be obtained in the co-cultivation step.

The term "labeling" denotes the presence or absence of a surface marker which can be determined by the addition of a specifically binding and labeled anti-surface marker antibody. Thus, the presence of a surface marker is determined e.g. in the case of a fluorescence label by the occurrence of a fluorescence whereas the absence of a surface marker is determined by the absence of a fluorescence after incubation with the respective specifically binding and labeled anti-surface marker antibody.

Different cell populations can be labeled by using different surface markers such as CD3⁺-cells (T-cells), CD19⁺-cells (B-cells), IgM⁺-cells (mature naive B-cells), IgG⁺-cells (mature B-cells), CD38⁺-cells (e.g. plasmablasts), and IgG⁺CD38⁺-cells (pre-plasma cells).

For a selection or enrichment of B-cells the cells are either single labeled, or double labeled, or triple labeled. Also required is a labeling that results in about 0.1 % to 2.5 % of labeled cells of the total cell population. In one embodiment B-cells are deposited as single cells selected by the labeling of surface molecules present on 0.1 % to 2.5 % of the B-cells in the population, in another embodiment on 0.3 % to 1.5 % of the B-cells of the population, in a further embodiment on 0.5 % to 1 % of the B-cells of the population.

The IgG⁺-B-cells within the PBMC population 0.5 % - 1 % can be doubly labeled as IgG⁺CD19⁺-cells, IgG⁺CD38⁺-cells, and IgG⁺CD268⁺-cells. Thus, in one embodiment of the use IgG⁺CD19⁺-B-cells, IgG⁺CD38⁺-B-cells, or IgG⁺CD268⁺-B-cells are deposited as single cells.

Of IgG⁻-B-cells within the PBMC population 0.5 % - 1 % can be doubly labeled as IgGCD138⁺-cells. Thus, in one embodiment of the use IgG⁻CD138⁺-B-cells are deposited as single cells.

The labeling of CD27⁺CD138⁺-cells or CD3⁻CD27⁺-cells results in about 1.5 % of the cells of the cell population to be labeled, respectively. Thus, in one embodiment of the use CD27⁺CD138⁺-B-cells or CD3⁻CD27⁺-B-cells are deposited as single cells.

Of IgG⁺-hamster-B-cells within the PBMC population 0.6 % ± 0.1 % can be doubly labeled as IgG⁺IgM⁻-hamster-B-cells. Thus, in one embodiment of the use IgG⁺IgM⁻-hamster-B-cells are deposited as single cells.

In one embodiment IgG⁻CD138⁺-B-cells are deposited as single cells from the B-cells obtained from an immunized animal. In one embodiment of the use IgG⁺CD19⁺-B-cells are deposited as single cells from the B-cells obtained from a non-immunized animal. In another embodiment of the use IgG⁺IgM⁻-B-cells are deposited as single cells from the B-cells obtained from a non-immunized or immunized animal. In one embodiment of the use IgG⁺CD19⁺-murine-B-cells are deposited as single cells. This selection step results in an improved or even the highest yield of IgG⁺-wells in the succeeding co-cultivation step. In another embodiment of the use IgG⁻CD138⁺-murine-B-cells are deposited as single cells. Therewith cells producing the highest amount of B-cell clones in the first place and secondly the highest concentration of IgG are selected (see Table 5). In another embodiment of the use IgG⁺CD19⁺-murine-B-cells and IgGCD138⁺-murine-B-cells are deposited as single cells. In one specific embodiment the use is with the proviso that if the cells are of rabbit origin the labeling is not of IgG⁺-B-cells and/or CD138⁺-B-cells.

IgG⁺-murine-B-cells can be labeled with the anti-mouse-IgG-antibody 227 (Ab 227), IgG⁺-hamster-B-cells can be labeled with the anti-hamster-IgG-antibody 213 (AB 213) and/or anti-hamster-IgG-antibody 225 (AB 225), and rabbit B-cells can be labeled with the anti-IgG-antibody 184 (see Table 4).

**Table 4: Immunofluorescence labeling of B-cells - the table present the average labeled fraction of the population of murine B-cells (A-E), hamster B-cells (F-H) and rabbit B-cells (I-J).**

| | **Single IgG labeling** | **IgG+CD19 labeling** | **IgG+IgM labeling** |
|---|---|---|---|
| A | IgG⁺ | - | IgG⁺IgM⁺ |
| | AB 185 PE | | AB 185 PE, AB 219 APC |
| | 17 % ± 3 % n=4 | | 12 % n= 1 |
| B | IgG⁺ | IgG⁺CD19⁺ | IgG⁺IgM⁺ |
| | AB 215 APC | AB 215 APC, AB 218 PE | AB 215 APC, AB 200 PE |
| | 12 % ± 3 % n=5 | 11 % n=1 | 14 % n=1 |
| C | IgG⁺ | IgG⁺CD 19⁺ | IgG⁺IgM⁺ |
| | AB 217 FITC | AB 217 FITC, AB 218 PE | AB 217 FITC, AB 200 PE |
| | 17 % ± 4 % n=7 | 10 % n=1 | 19 % n= 1 |
| D | IgG⁺ | IgG⁺CD19⁺ | IgG⁺IgM⁺ |
| | AB 222 FITC | AB 222 FITC, AB 218 PE | AB 222 FITC, AB 200 PE |
| | 18 % ± 2 % n=3 | 15 % n=1 | 14 % n= 1 |
| E | IgG⁺ | IgG⁺CD19⁺ | IgG⁺IgM⁺ |
| | AB 227 FITC | AB 227 FITC, AB 218 PE | AB 227 FITC, AB 200 PE |
| | 0.8 % ± 0.3 % n=13 | 0.5 % n=1 | 0.2 % n= 1 |
| F | IgG⁺ | no B-cell marker known | IgG⁺IgM⁺ |
| | AB 212 FITC | | AB 212 FITC, AB 223 APC |
| | 43 % ± 6 % n=7 | | 43 % n= 1 |
| G | IgG⁺ | no B-cell marker known | IgG⁺IgM⁺ |
| | AB 213 APC | | AB 213 APC, AB 224 FITC |
| | 0.9 % ± 0.4 % n=27 | | 0.07 % n= 1 |
| H | IgG⁺ | no B-cell marker known | IgG⁺IgM⁺ |
| | AB 225 PE | | AB 225 PE, AB 224 FITC |
| | 17 % ± 3 % n=5 | | 0.7 % n= 1 |
| I | IgG⁺ | - | - |
| | AB 120 PE | | |
| | > 10% | | |
| J | IgG⁺ | - | - |
| | AB 184 FITC | | |
| | 0.3 - 2 % | | |

| | | |
|---|---|---|
| AB 120 - goat anti-rabbit IgG-antibody | Southern Biotech | 4030-09 |
| AB 184 - goat anti-rabbit IgG Fc-antibody | AbDSerotech | STAR121F |
| AB 185 - goat anti-mouse IgG-antibody | Caltag | M3 5004-3 |
| AB 200 - goat anti-mouse IgM-antibody | Invitrogen | M31504 |
| AB 212 - goat anti-hamster IgG-antibody | AbDSerotech | STAR79F |
| AB 213 - mouse anti-hamster IgG-antibody | Becton Dickinson | 554010 |
| AB 215 - goat anti-mouse IgG-antibody | Sigma | B 0529 |
| AB 217 - goat anti-mouse IgG-antibody | AbDSerotech | STAR120F |
| AB 218 - rat anti-mouse CD 19-antibody | Abcam | ab22480 |
| AB 219 - goat anti-mouse IgM-antibody | Rockland | 710-1607 |
| AB 222 - goat anti-mouse IgG-antibody | Abcam | ab7064 |
| AB 223 - mouse anti-hamster IgM-antibody | Becton Dickinson | 554035 |
| AB 224 - mouse anti-hamster IgM-antibody | Becton Dickinson | 554033 |
| AB 225 - mouse anti-hamster IgG-antibody | Becton Dickinson | 554056 |
| AB 227 - goat anti-mouse IgG-antibody | Sigma | F 8264 |

PE: Phycoerythrin
APC: Allophycocyanin
FITC: Fluorescein isothiocyanate

It has to be pointed out that not all commercially available antibodies can be used for the labeling due to their low or non existing specificity.

Murine-B-cells can be labeled with the anti-IgG-antibody 227, hamster-B-cells can be labeled with the anti-IgG-antibody 213.

IgG⁺CD19⁺-murine-B-cells can be labeled with antibody 227 and antibody 218,
IgG⁺IgM⁻-murine-B-cells can be labeled with antibody 227 and antibody 219,
IgG⁺IgM⁻-hamster-B-cells can be labeled with antibody 213 and antibody 224,
IgG⁺-rabbit-B-cells can be labeled with antibody 184,
IgG⁺IgM⁻-rabbit-B-cells can be labeled with antibody 184 and antibody 254 and SA 263,
IgG⁺CD138⁺-rabbit-B-cells can be labeled with antibody 259 and antibody 256.

Murine B-cells can be labeled with the anti-CD27 antibody 235 or 236 (AB 235, AB 236), the anti-CD38 antibody 192 (AB 192), the anti-CD138 antibody 233 (AB 233) and the anti-CD268 antibody 246 (AB 246).

**Table 5: Immuno fluorescence labeling for the determination of mature mouse-(A-J), hamster- (K) and rabbit (L-N)-B-cells.**

| **labeling** | **Immuno fluorescence labeling for sorting of B-cells** | **Percentage of all viable cells %** |
|---|---|---|
| A | IgG⁺CD19⁺ - AB 227 FITC, AB 218 PE | 0.5 ± 0.2 n=14 |
| B | IgG⁺CD38⁺ - AB 227 FITC, AB 192 PE | 0.8 ± 0.5 n= 9 |
| C | IgG⁺CD138⁺ - AB 227 FITC, AB 233 PE | 0.06 ± 0.07 n= 6 |
| D | IgG⁻CD138⁺ - AB 227 FITC, AB 233 PE | 0.6 ± 0.5 n=6 |
| E | IgG⁺CD27⁺ - AB 227 FITC, AB 235 PE | 0.1 ± 0.1 n= 8 |
| F | CD27⁺CD138⁺ - AB 236 A647, AB 233 PE | 1.5 ± 0.5 n= 2 |
| G | CD27⁺IgG⁺CD3⁻ - AB 235 PE, AB 227 FITC, AB 241 A647 | 0.10 ± 0.04 n= 3 |
| H | CD3⁻CD27⁺ - AB 189 FITC, AB 235 PE | 1.33 n= 1 |
| I | IgG⁺CD268⁺ - AB 227 FITC, AB 246 A647 | 0.8 n= 1 |
| J | CD38⁺CD3⁻ - AB 192 PE, AB 189 FITC | 12 ± 7 n= 2 |
| K | IgG⁺IgM⁻ - AB 213 A647, AB 224 FITC | 0.6 ± 0.1 n= 15 |
| L | IgG⁺ - AB 184 FITC | 0.6 ± 0.2, n= 5 |
| M | IgG⁺IgM⁻ - AB 184 FITC, AB 254 Biotin, SA 263 PE | 0.4 ± 0.2, n=2 |
| N | IgG⁺CD138⁺ - AB 259, AB 256 PE | 0.3 ± 0.1, n= 5 |

| | | |
|---|---|---|
| AB 184 - goat anti-rabbit IgG-antibody | AbD Serotec | STAR121F |
| AB 189 - hamster anti-mouse CD3-antibody | Becton Dickinson | 553062 |
| AB 192 - rat anti-mouse CD38-antibody | Becton Dickinson | 553764 |
| AB 213 - mouse anti-hamster IgG-antibody | Becton Dickinson | 554010 |
| AB 218 - rat anti-mouse CD 19-antibody | Abcam | ab22480 |
| AB 224 - mouse anti-hamster IgM-antibody | Becton Dickinson | 554033 |
| AB 227 - goat anti-mouse IgG-antibody | Sigma | F 8264 |
| AB 233 - rat anti-mouse CD138-antibody | Becton Dickinson | 553714 |
| AB 235 - hamster anti-mouse CD27-antibody | Becton Dickinson | 558754 |
| AB 236 - hamster anti-mouse CD27-antibody | Becton Dickinson | 558753 |
| AB 241 - hamster anti-mouse CD3-antibody | Becton Dickinson | 553060 |
| AB 246 - rat anti-mouse BAFF-R-antibody | eBioscience | 51-5943 |
| AB 254 - mouse anti-rabbit IgM-antibody | Becton Dickinson | custom made |
| AB 256 - goat anti-rat IgG-antibody | Southern Biotech | 3030-09 |
| AB 259 - rat anti-rabbit CD138-antibody | Roche Glycart AG | |
| SA 263 - Streptavidin | Invitrogen | S866 |

A647: Alexa Fluor® 647
FITC: Fluorescein isothiocyanate

In one embodiment the use comprises the step of depleting the B-cell population of macrophages and enriching of B-cells of the B-cell population secreting antibody specifically binding a target antigen.

### Single cell depositing:

The use according to the invention comprises the step of depositing the B-cells of a B-cell population as single cells. In one embodiment of the use the depositing as single cells is by fluorescence activated cell sorting (FACS). The labeling required for the FACS single cell depositing can be carried out as reported in the previous section.

In one embodiment of the use specifically labeled B-cells are deposited as single cells. In a further embodiment of the use the labeling is a labeling of cell surface markers with fluorescence labeled antibodies. In another embodiment the use provides for monoclonal antibodies. In one embodiment of the use mature B-cells are deposited as single cells.

It has also been found that an additional centrifugation step after the single cell depositing and prior to the co-cultivation provides for an increased number of antibody secreting cells and increases the amount of the secreted IgG (example experimental animal with human immunoglobulin locus, see Table 6).

**Table 6: IgG positive wells/cell clones with and without centrifugation step after single cell depositing.**

| **huCk ELISA** | **with centrifugation step** | **without centrifugation step** |
|---|---|---|
| huCk⁺ wells [n] | 9 | 1 |
| huCk⁺ wells [% total wells] | 13 | 1 |
| huCk conc. of all huCk⁺ wells [average ng/ml] | 76.4 | 9.7 |

In one embodiment of the use the use comprises the step of centrifuging the single deposited cells prior to the co-cultivation. In one specific embodiment the centrifuging is for 5 min. at 300 x g.

### Co-cultivation:

The co-cultivation step with feeder cells can be preceded and also succeeded by a number of additional steps.

In one embodiment of the use the single deposited B-cells are co-cultivated with feeder cells in the presence of a feeder mix. In a specific embodiment the B-cells are co-cultivated with murine EL-4 B5 feeder cells. By suitable immuno fluorescence labeling as outlined above an increase in the yield in the co-cultivation step (number of IgG⁺-wells/cell clones as well as IgG-concentration) and also an enrichment or isolation of mature IgG⁺-B-cell from PBMCs can be achieved.

With the single cell depositing of IgG⁺CD19⁺- and/or IgG⁺CD38⁺-B-cells from freshly isolated PBMCs the highest number of IgG⁺-wells/cell clones can be obtained. With the single cell depositing of IgG⁺CD19⁺-, IgG⁺CD38⁺- and/or IgG⁻CD138⁺-B-cells after the depletion of macrophages or KLH-specific cells (keyhole limpet haemocyanine) good results can be obtained. With the single cell depositing of IgG⁺CD19⁺-, IgG⁺CD38⁺- and/or IgG⁻CD138⁺-B-cells after the depletion of antigen-specific B-cells improved results can be obtained. Thus, in one embodiment of the use IgG⁺CD19⁺-, IgG⁺CD38⁺- and/or IgG⁻CD138⁺-B-cells are deposited as single cells.

It has been found that a single cell depositing based on a labeling as outlined above results in the highest fraction of IgG⁺-wells/cell clones and in the wells/cell clones with the highest IgG-concentration in the supernatant. Thus, in one embodiment of the use IgG⁺CD19⁺- and/or IgG⁻CD138⁺-murine-B-cells are deposited as single cells. In one embodiment of the use IgG⁺IgM⁻-hamster-B-cells are deposited as single cells. In one embodiment of the use IgG⁺-, and/or IgG⁺CD138⁺-, and/or CD138⁺- and/or IgG⁺IgM⁻-rabbit-B-cells are deposited as single cells.

**Table 7: Yield in the co-cultivation depending on the immuno fluorescence labeling.**

| **labeling** | | **nₜₒₜₐₗ wells** | **IgG⁺-wells of nₜₒₜₐₗ wells (%)** | | | **average IgG-concentration (ng/ml)** | | |
|---|---|---|---|---|---|---|---|---|
| | | **isol/depl/enr** | isol. | depl. | enr. | isol. | depl. | enr. |
| mouse | IgG⁺CD19⁺ | 356/356/324 | 45 | 50 | 37 | 68 | 46 | 42 |
| | IgG⁺ | -/144/144 | - | 32 | 7 | - | 34 | 31 |
| | IgG⁺CD38⁺ | 72/190/190 | 36 | 41 | 43 | 37 | 26 | 27 |
| | IgG⁺CD138⁺ | 72/72/72 | 3 | 13 | 12 | 22 | 59 | 43 |
| | IgG⁻CD138⁺ | 36/108/48 | 19 | 52 | 37 | 55 | 31 | 51 |
| | IgG⁺CD27⁺ | 64/64/64 | 4 | 28 | 20 | 102 | 54 | 32 |
| | CD27⁺CD138⁺ | -/32/- | - | 6 | - | - | 135 | - |
| | CD27⁺IgG⁺CD3⁻ | 72/72/72 | 14 | 0 | 14 | 4 | 0 | 0 |
| | CD3⁻CD27⁺ | -/32/- | - | 13 | - | - | 29 | - |
| hamster | IgG⁺CD268⁺ | -/72/- | - | 35 | - | - | 93 | - |
| | IgG⁺IgM⁻ | -/216/216 | - | 17 | 22 | - | 78 | 93 |
| | IgG⁺ | -/216/216 | - | 10 | 35 | 1 | 71 | 64 |
| rabbit | IgG⁺ | -/1512/1307 | - | 33 | 28 | - | 59 | 60 |
| | IgG⁺IgM⁻ | -/76/- | - | 29 | - | - | 5 | - |
| | CD138⁺ | -/2016/- | - | 14 | - | - | 16 | - |
| | IgG⁺CD138⁺ | -/168/- | - | 37 | - | - | 64 | - |

For murine B-cells with the single cell depositing of IgG⁺CD19⁺-cells after each enrichment (enr.) and/or depletion (depl.) step the highest number of IgG⁺-wells/cell clones after co-cultivation can be obtained. Alternatively, with the single cell depositing of IgGCD138⁺-cells wells/cell clones with the best IgG-concentration in the supernatant can be obtained. The single cell depositing of IgG⁻CD138⁺-cells can be used for B-cells from immunized animals. The single cell depositing of IgG⁺CD19⁺-cells can be used for B-cells from non-immunized animals. The single cell depositing of IgG⁺IgM⁻-cells can be used for hamster-B-cells of immunized and non-immunized animals. The single cell depositing of IgG⁺-, and/or IgG⁺CD138⁺-, and/or CD138⁺- and/or IgG⁺IgM⁻-B-cells can be used for rabbit-B-cells.

The immuno fluorescence labeling used for B-cells obtained from the blood of an experimental animal can also be used for the labeling of B-cells obtained from the spleen and other immunological organs of an experimental animal, such as mouse, hamster and rabbit. For mouse B-cells the fraction of IgG⁺-B-cells from spleen was about 0.8 % compared to 0.4 % for IgG⁺CD19⁺-cells. For hamster B-cells the respective numbers are 1.9 % and 0.5 % IgG⁺IgM⁻-cells. For rabbit-blood derived B-cells 0.2 % of IgG⁺-cells were found after depletion of macrophages. Peyer'sche plaques from rabbit showed 0.4 % of IgG⁺-cells and spleen showed 0.3 % of IgG⁺-cells after depletion of macrophages.

With the use according to the invention after about seven (7) days, i.e. after 5, 6, 7, or 8 days, especially after 7 or 8 days, of co-cultivation antibody concentrations of from about 30 ng/ml up to 15 µg/ml or more can be obtained (average value about 500 ng/ml). With the thereby provided amount of antibody a high number of different analyses can be performed in order to characterize the antibody, e.g. regarding binding specificity, in more detail. With the improved characterization of the antibody at this early stage in the screening / selection process it is possible to reduce the number of required nucleic acid isolations and sequencing reactions that have to be performed. Additionally the B-cell clone provides an amount of mRNA encoding monoclonal light and heavy chain variable region allowing the use of degenerated PCR primer and obviates the requirement of highly specific primer. Also the required number of PCR cycles is reduced. Thus, in one embodiment the reverse transcriptase PCR is with degenerated PCR primer for the light and heavy chain variable domain.

It is especially suited to use the thymus gland of young animals compared to the isolation of thymocytes from the blood adult animals. The term "young animal" denotes an animal before sexual maturity occurs. A young hamster, for example, is of an age of less than 6 weeks, especially less than 4 weeks. A young mouse, for example, is of an age of less than 8 weeks, especially less than 5 weeks.

Due to the origin of the feeder mix, which is derived from the supernatant of cultivated thymocytes (thymocyte cultivation supernatant - TSN), considerable batch to batch variations occur. In order to overcome this variability a synthetic feeder mix consisting of synthetic components has been developed. A feeder mix consisting of IL-1β (interleukin-1 beta), TNFα (tumor necrosis factor alpha), IL-2 (interleukin-2) and IL-10 (interleukin-10) is known from Tucci, A., et al., J. Immunol. 148 (1992) 2778-2784.

The current invention comprises a synthetic feeder mix for the co-cultivation of single deposited B-cells and feeder cells. The invention also comprises B-cell-species-specific additives for the synthetic feeder mix for increasing the amount of secreted antibody by the respective B-cell clone. Concomitantly highly producing cells contain more mRNA which in turn facilitates the reverse transcription and sequencing of the encoding nucleic acid, e.g. with a redundant, non-specific primer set.

By the addition of SAC (Staphylococcus aureus strain Cowans cells, a single SAC lot was used) the number of antibody secreting B-cells and the average IgG-concentration in the supernatant after co-cultivation can be increased. It has been found that for the addition of SAC in the co-cultivation a concentration range can be defined as reduced as well as increased concentrations of SAC reduce the amount of secreted antibody.

**Table 8a: Results of a huCk ELISA (huCk = human C kappa) or rbIgG ELISA of cell culture supernatants of B-cells obtained from an experimental animal with human IgG locus or a wildtype rabbit (NZW) co-cultivated with EL-4 B5 feeder cells and TSN as feeder mix with or without added SAC.**

| | | | | | |
|---|---|---|---|---|---|
| | **TSN** | **TSN + SAC** | | | |
| huCk⁺ wells [n] | 7 | 45 | | | |
| huCk⁺ wells [% total wells] | 5 | 31 | | | |
| huCk conc. of all huCk⁺ wells [Ø ng/ml] | 89.1 | 41.0 | | | |

| | SAC | SAC | SAC | SAC | |
|---|---|---|---|---|---|
| | 1:5000 | 1:10000 | 1:20000 | 1:40000 | |
| rbIgG⁺ wells [n] | 13 | 15 | 27 | 30 | |
| rbIgG⁺ wells [% total wells] | 15 | 18 | 32 | 36 | |
| rbIgG conc. of all rbIgG⁺ wells [Ø ng/ml] | 149.0 | 159.1 | 233.7 | 197.2 | |

| | **w/o** | **SAC 1:20000** | **SAC 1:50000** | **SAC 1:100000** | **SAC 1:150000** |
|---|---|---|---|---|---|
| rbIgG⁺ wells [n] | 12 | 75 | 93 | 92 | 72 |
| rbIgG⁺ wells [% total wells] | 5 | 30 | 37 | 37 | 29 |
| rbIgG conc. of all rbIgG⁺ wells [Ø ng/ml] | 199 | 665 | 742 | 774 | 668 |

It can be seen that a SAC ratio of from 1:20000 to 1:150000 provides for an increased number of IgG⁺-wells/cell clones, whereby the ratio of from 1:50000 to 1:100000 shows the highest numbers. In one embodiment the amount of SAC added to the cultivation medium is determined by providing a dilution series and determining the dilution at which the added SAC provides for the highest number of IgG positive wells/cell clones.

It has been observed that by the addition of SAC to the feeder-mix the co-cultivation of B-cells was surprisingly changed in such a way that only single deposited B-cells have a benefit in growth, whereas B-cell growth was inhibited when using a PBL (e.g. B cells and endogenous T cells) mixture for co-cultivation.

**Table 8b: Results of a huCk ELISA or rbIgG ELISA of cell culture supernatants of PBLs and single deposited B-cells co-cultivated with EL-4 B5 feeder cells and TSN as feeder mix with added SAC.**

| **rbIgG ELISA** | **PBLs* (30 cells)** | **single deposited rbIgG⁺-B-cell** |
|---|---|---|
| rbIgG⁺ wells [n] | 8 | 104 |
| rbIgG⁺ wells [% total wells] | 6 | 58 |
| rbIgG conc. of all huCk⁺ wells [average ng/ml] | 55.0 | 129.2 |

| | | |
|---|---|---|
| * depleted of macrophages | | |

Further data obtained with different feeder mixes is presented in the following Tables 9 and 10.

In one specific embodiment IL-1β, TNFα, IL-2, and IL-10 are recombinant murine IL-1β, murine TNFα, murine IL-2, and murine IL-10.

In one embodiment of the use the synthetic feeder mix for the co-cultivation of hamster B-cells comprises IL-1β, IL-2, IL-10, TNF-α, IL-6 and SAC. In one specific embodiment IL-6 is added at a concentration of 10 ng/ml. In one specific embodiment SAC is added at a 1:75,000 ratio.

**Table 9: Results of an rbIgG ELISA of cell culture supernatants of rabbit B-cells co-cultivated with EL-4 B5 feeder cells and different synthetic feeder mixes comprising recombinant murine substances in different combinations.**

| | **rabbit TSN, SAC** | **IL-6, IL-1β, TNFα, IL-2, IL-10** | **IL-6, TNFα, IL-2, IL-10** | **IL-6, IL-1β, IL-2, IL-10** | **IL-6, IL-1β, TNFα, IL-2** | **IL-6, IL-1β, TNFα, IL-10** | **IL-1β, TNFα, IL-2, IL-10** |
|---|---|---|---|---|---|---|---|
| rbIgG⁺ wells [n] | 37 | 24 | 12 | 16 | 18 | 23 | 24 |
| rbIgG⁺ wells [% total wells] | 51 | 33 | 17 | 22 | 25 | 32 | 33 |
| rbIgG conc. of all rbIgG⁺ wells [Ø ng/ml] | 196.0 | 289.9 | 32.4 | 75.7 | 166.4 | 134.4 | 203.6 |

**Table 10: IgG⁺-wells of cell culture supernatants of rabbit B-cells co-cultivated with EL-4 B5 feeder cells and TSN or a feeder mix comprising recombinant murine substances and SAC (rb = rabbit, m = mouse).**

| rbIgG⁺ wells [n] | **TSN + SAC** | **IL-1β, TNFα, IL-2, IL-10 + SAC** |
|---|---|---|
| pure | 64 | 55 |
| + mIL21 | 22 | 25 |
| + mIL10 | 78 | 61 |
| + mIL21+ mIL10 | 57 | 93 |

| rbIgG⁺ wells [% total wells] | | |
|---|---|---|
| pure | 25 | 22 |
| + mIL21 | 9 | 10 |
| + mIL10 | 31 | 24 |
| + mIL21+ mIL10 | 23 | 37 |

| rbIgG conc. of all rbIgG⁺ wells [Ø ng/ml] | | |
|---|---|---|
| pure | 312.3 | 662.3 |
| + mIL21 | 263.7 | 541.1 |
| + mIL10 | 553.0 | 522.3 |
| + mIL21+ mIL10 | 422.6 | 307.5 |

A co-cultivation of feeder cells and murine B-cells without IL-2, without IL-10, as well as without IL-2 and IL-10 results in an increase in the yield of IgG⁺-wells albeit the IgG-concentration is reduced. Without TNFα the IgG-concentration is also reduced. Without IL-1β no IgG can be found in the supernatant.

A co-cultivation of hamster B-cells without IL-2 or without IL-10, respectively, results in IgG⁺-wells with detectable IgG-concentration. In contrast thereto in a co-cultivation without IL-2 and IL-10 almost no B-cell growth can be detected. In the absence of TNF-α or IL-1β no IgG-secretion can be determined.

In the presence of EL-4 B5 feeder cells at least IL-1β and TNFα are required for the co-cultivation of mouse, hamster and rabbit B-cells. IL-2 and IL-10 can be omitted for the co-cultivation of murine cells. Hamster B-cells can be cultivated in the absence of either IL-2 or IL-10. Rabbit B-cells can be cultivated in the absence of either IL-2 or IL-10 or IL-6.

For murine and hamster B-cells the addition of IL-4 to the feeder mix increases the number of IgG⁺-wells/cell clones as well as the IgG-concentration in the supernatant.

The addition of IL-6 to the feeder mix for the co-cultivation of murine-B-cells or hamster-B-cells results in an increased number of IgG⁺-wells/cell clones or increased IgG-concentration, respectively. Thus, in one embodiment of the use the feeder mix for the co-cultivation of murine-B-cells or hamster-B-cells comprises IL-6. In one specific embodiment the IL-6 is added at a concentration of 50 ng/ml. In one specific embodiment IL-6 is added at a concentration of 10 ng/ml, if high IgG-concentration is required. In one specific embodiment the addition of IL-6 is after three days of co-cultivation of the selected B-cells and EL-4 B5 cells.

One aspect of the invention is a synthetic feeder mix for the co-cultivation of B-cells and feeder cells that comprises IL-1β, TNFα, IL-10, and one or more selected from SAC, IL-2, and IL-6.

One aspect of the invention is a synthetic feeder mix for the co-cultivation of B-cells and feeder cells that comprises IL-1β, TNFα, IL-2, IL-10 and SAC.

One aspect of the invention is a synthetic feeder mix for the co-cultivation of murine B-cells and feeder cells that is consisting of IL-1β, TNFα, and optionally comprises IL-21, and/or SAC, and/or BAFF, and/or IL-6.

One aspect of the invention is a synthetic feeder mix for the co-cultivation of murine or hamster B-cells and feeder cells that comprises IL-1β, TNFα, IL-2, IL-10 and IL-6

One aspect of the invention is a synthetic feeder mix for the co-cultivation of hamster B-cells and feeder cells that is consisting of IL-1β, TNFα, and IL-2 or IL-10, and SAC.

One aspect of the invention is a synthetic feeder mix for the co-cultivation of hamster B-cells and feeder cells comprises IL-1β, IL-2, IL-10, TNF-α, IL-6 and SAC.

One aspect of the invention is a synthetic feeder mix for the co-cultivation of rabbit B-cells and feeder cells that comprises IL-1β, TNFα, IL-10, and IL-6.

One aspect of the invention is a synthetic feeder mix for the co-cultivation of rabbit B-cells and feeder cells that comprises IL-1β, TNFα, IL-10, IL-6 or IL-2, and SAC

In one specific embodiment IL-1β, TNFα, IL-2, and IL-10 are recombinant murine IL-1β, murine TNFα, murine IL-2, and murine IL-10.

In one specific embodiment IL-6 is added at a concentration of 10 ng/ml.

In one specific embodiment SAC is added at a 1:75,000 ratio.

In one specific embodiment the feeder cells are murine EL-4 B5 cells.

The addition of an inhibitor of a certain potassium channel (= PAP-1, 5-(4-phenoxy butoxy) psoralene) surprisingly increases the rbIgG secretion of B-cells in a concentration dependent manner without decreasing the number of B-cell clones. Usually a cytokine which induced rbIgG productivity can be correlated with a decrease of the overall number of B-cell clones. This was not the case with PAP-1.

**Table 11: Results of an rbIgG ELISA of cell culture supernatants of B-cells co-cultivated with EL-4 B5 feeder cells in the presence of TSN and SAC (=w/o) and different concentrations of PAP-1. DMSO: solvent for PAP-1 (1 µM).**

| | **w/o** | **0.01 µM** | **0.1 µM** | **1 µM** | **10 µM** | **DMSO** |
|---|---|---|---|---|---|---|
| rbIgG⁺ wells [n] | 53 | 72 | 69 | 93 | 80 | 76 |
| rbIgG⁺ wells [% total wells] | 21 | 29 | 27 | 37 | 32 | 30 |
| rbIgG conc. of all huCk⁺ wells [average ng/ml] | 195.8 | 289.0 | 452.9 | 579.5 | 890.7 | 225.3 |

With a TSN concentration of 7.5 % the highest IgG concentration in the supernatant can be obtained.

**Table 12: Influence of TSN on co-cultivation. A TSN concentration of 7.5 % results in improved B-cell growth and productivity**

| | **5% TSN** | **7.5% TSN** | **10% TSN** |
|---|---|---|---|
| rbIgG⁺ wells [n] | 71 | 71 | 81 |
| rbIgG⁺ wells [% total wells] | 28 | 28 | 32 |
| rbIgG conc. of all rbIgG⁺ wells [Ø ng/ml] | 246 | 512 | 372 |

With a number of 30,000 feeder cells per well of a 96-well plate the highest number of IgG⁺-wells in combination with IgG concentration in the supernatant can be obtained. In one embodiment of the use the number of feeder cells per single deposited B-cell is about 30,000.

**Table 13: Influence of the amount of EL-4 B5 feeder cells on co-cultivation.**

| | **20000** | **22000** | **24000** | **30000** | **35000** | **40000** |
|---|---|---|---|---|---|---|
| rbIgG⁺ wells [n] | 71 | 73 | 78 | 78 | 73 | 38 |
| rbIgG⁺ wells [% total wells] | 28 | 29 | 31 | 31 | 29 | 15 |
| rbIgG conc. of all rbIgG⁺ wells [Ø ng/ml] | 246 | 319 | 346 | 418 | 457 | 656 |

The co-cultivation is in one embodiment of the use in polystyrene multi well plates with wells with a round bottom. The working volume of the wells is in one embodiment of the use of 50 µl to 250 µl. In one specific embodiment the wells are coated at least partially with a non-fibrous substrate prepared from a blend of polymer plastic resin and amphipathic molecules, wherein the amphipathic molecule comprises a hydrophilic moiety and a hydrophobic region, wherein the hydrophobic regions are anchored within the substrate and the hydrophilic moieties are exposed on the substrate. In one specific embodiment the amphipathic molecules are chosen from alkylamine ethoxylated, poly (ethylene imine), octyldecamine or mixtures thereof (see e.g. EP 1 860 181).

### Characterization of co-cultivated cells:

For the (qualitative and quantitative) determination of secreted IgG after the co-cultivation generally all methods known to a person of skill in the art such as an ELISA can be used. For the determination of IgG secreted by murine B-cells an ELISA with the anti-IgG antibodies AB 216 (capture antibody) and AB 215 (tracer antibody) is used. For the determination of IgG secreted by hamster B-cells an ELISA with the monoclonal antibodies AB 220 (capture antibody) and AB 213 (tracer antibody) is used.

Depending on the characterization results a B-cell clone can be obtained, i.e. selected. The term "clone" denotes a population of dividing and antibody secreting B-cells arising from/originating from a single B-cell. Thus, a B-cell clone produces a monoclonal antibody.

### Isolation of mRNA, cloning and sequencing:

From the B-cells the total mRNA can be isolated and transcribed in cDNA. With specific primers the cognate VH- and VL-region encoding nucleic acid can be amplified. With the sequencing of the therewith obtained nucleic acid it was confirmed that the obtained antibodies are monoclonal antibodies in most cases (71-95 %). Also can be seen from the sequencing of the individual B-cells that almost no identical sequences are obtained. Thus, the method provides for highly diverse antibodies binding to the same antigen.

The primers used for the amplification of the VH-encoding nucleic acid can be used for cDNA obtained from cells from the NMRI-mouse, the Armenian Hamster, the Balb/c-mouse as well as the Syrian hamster and the rabbit.

The amino acid sequence is derived from the amplified VH-encoding nucleic acid and the exact start and end point is identified by locating the amino acid sequences of EVQL/QVQL to VSS (VH-region) and DIVM/DIQM to KLEIK (VL-region).

The term "antibody" denotes a protein consisting of one or more polypeptide chain(s) substantially encoded by immunoglobulin genes. The recognized immunoglobulin genes include the different constant region genes as well as the myriad immunoglobulin variable region genes. Immunoglobulins may exist in a variety of formats, including, for example, Fv, Fab, and F(ab)₂ as well as single chains (scFv), diabodies, monovalent, bivalent, trivalent or tetravalent forms, and also as bispecific, trispecific or tetraspecific form (e.g. Huston, J.S., et al., Proc. Natl. Acad. Sci. USA 85 (1988) 5879-5883; Bird, R.E., et al., Science 242 (1988) 423-426; in general, Hood et al., Immunology, Benjamin N.Y., 2nd edition (1984); and Hunkapiller, T. and Hood, L., Nature 323 (1986) 15-16).

An "expression cassette" refers to a construct that contains the necessary regulatory elements, such as promoter and polyadenylation site, for expression of at least the contained nucleic acid in a cell.

The term "experimental animal" denotes a non-human mammal. In one embodiment the experimental animal is selected from rat, mouse, hamster, rabbit, non-human primates, sheep, dog, cow, chicken, amphibians, and reptiles.

The following examples are provided to aid the understanding of the present invention, the true scope of which is set forth in the appended claims.

### Examples

### Example 1

### Media and buffers:

Blocking buffer for ELISA comprises 1X PBS and 1% BSA.

Coating buffer for ELISA comprises 4.29g Na2CO3* 10 H2O and 2.93g NaHCO3 add water to a final volume of 1 liter, pH 9.6 adjusted with 2 N HCl.

Ethanol-solution for RNA isolation comprises 70 % Ethanol or 80 % Ethanol.

FACS-buffer for immuno fluorescence staining comprises 1X PBS and 0.1 % BSA. IMDM-buffer for ELISA comprises 1X PBS, 5 % IMDM and 0.5 % BSA.

Incubation buffer 1 for ELISA comprises 1X PBS, 0.5 % CroteinC.

Incubation buffer 2 for ELISA comprises 1X PBS, 0.5 % CroteinC and 0.02 % Tween 20.

Incubation buffer 3 for ELISA comprises 1X PBS, 0.1 % BSA.

Incubation buffer 4 for ELISA comprises 1X PBS, 0.5 % BSA, 0.05 % Tween, PBS (10X), 0.01 M KH2PO4, 0.1 M Na2HPO4, 1.37 M NaCl, 0.027 M KCl, pH 7.0.

PCR-buffer comprises 500 mM KCl, 15 mM MgCl2, 100 mM Tris/HCl, pH 9.0.

Wash buffer 1 for ELISA comprises 1X PBS, 0.05 % Tween 20.

Wash buffer 2 for ELISA comprises 1X PBS, 0.1 % Tween 20.

Wash buffer 3 for ELISA comprises water, 0.9 % NaCl, 0.05 % Tween 20.

EL-4 B5 medium comprises RPMI 1640, 10 % FCS, 1 %

Glutamin/Penicillin/Streptomycin-Mix, 2 % 100 mM sodium pyruvate, 1 % 1 M HEPES buffer.

### Example 2

### Animal care and immunization

The experimental animals were held according to the German animal protection law (TierSCHG) as well as according to the respective European guidelines.

Mice and hamster were received at an age of from 6 to 8 weeks and were immunized prior to an age of 12 weeks. The antigen was at first applied together with complete Freud's adjuvant (CFA). Further applications were with incomplete Freud's adjuvant (IFA). The antigen containing emulsion was applied subcutaneously whereby the emulsion comprised an amount of from 50 to 100 µg antigen depending on the weight of the receiving experimental animal.

NZW rabbits (Charles River Laboratories International, Inc.) were used for immunization. The antigen was solved in K₃PO₄ buffer pH 7.0 at a concentration of 1 mg/ml and mixed (1:1) with complete Freud's adjuvant (CFA) till generation of stabile emulsion. The rabbits received an intra dermal (i.d.) injection of 2 ml of emulsion followed by a second intra muscular (i.m.) and third subcutaneous (s.c.) injection each with 1 ml in one week interval. The fourth i.m. injection of 1 ml was performed two weeks later followed by two further s.c. injections of 1 ml in four weeks interval.

During the immunization serum antibody titer was determined with an antigen specific assay. At an antibody titer with an IC₅₀ of 1:10000 the blood or the spleen of the immunized animal was removed. For reactivation of antigen specific B-cells 30 µg to 50 µg of the antigen was applied intravenously to the experimental animal three days prior to the removal of the blood or the spleen.

### Example 3

### Removal of organs, blood and macrophages

Blood from mice and hamster was obtained by punctuation of the retrobulberic vein. Blood from rabbits was obtained by punctuation of the ear vein or, for larger volumes, of the ear artery. Whole blood (10 ml) was collected from rabbits 4-6 days after the third, fourth, fifth and sixth immunization and used for single cell sorting by FACS.

Macrophages were isolated from the obtained blood by attachment to cell culture plastic. From mice and hamsters, about 3^{∗}10⁵ macrophages can be obtained from each animal by this method.

If a larger amount of mouse or hamster macrophages was required, peritoneal macrophages were isolated. For this the animals have to be at least 3 months of age. For the removal of peritoneal macrophages, animals were sacrificed and 5 ml of EL-4 B5 medium with a temperature of 37 °C was immediately injected into the peritoneal cavity. After kneading the animal's belly for 5 minutes, the solution containing the cells was removed.

### Example 4

### Cultivation of EL-4 B5 cells

The frozen EL-4 B5 cells were thawed rapidly in a water bath at 37 °C and diluted with 10 ml EL-4 B5 medium. After centrifugation at 300 x g for 10 minutes the supernatant was discarded and the pellet resuspended in medium. After a further centrifugation step the supernatant was discarded again and the pellet was resuspended in 1 ml medium.

The EL-4 B5 cells were inoculated at a cell density of 3 x 10⁴ cells/ml in 175 m² cultivation flasks. Cell density was determined every second day and adjusted to 3 x 10⁴ cell/ml. The cells have a doubling time of approximately 12 hours and have to be cultivated at a cell density below 5 x 10⁵ cell/ml because with higher cell density the stimulatory properties of the cells are lost.

When the total cell number was about 1.5 x 10⁹ cells the medium was removed by centrifugation. Afterwards the cells were irradiated with 50 gray (5000 rad). After the determination of the viable cell number by trypan blue staining between 5 x 10⁶ and 1 x 10⁷ cells are aliquoted and frozen at -80 °C.

For co-cultivation the cells were thawed and washed twice with EL-4 B5 medium. For determination of the viable cell number the cell suspension is diluted 1:10 with 0.4 % (w/v) trypan blue solution and 10 µl of the mixture is transferred to a Neubauer counting chamber and cell number was counted.

### Example 5

### Density gradient centrifugation

The isolation of peripheral blood mononuclear cells (PBMCs) was effected by density gradient separation with Lympholyte® according to manufacturer's instructions A (Lympholyte®-mammal, cedarlane).

Withdrawn blood was diluted 2:1 with phosphate buffered saline (PBS). In a centrifuge vial the same volume of density separation medium was provided and the diluted blood is carefully added via the wall of the vial. The vial was centrifuged for 20 min. at 800 x g without braking. The lymphocytes were obtained from the white interim layer. The removed cells were supplemented with 10 ml PBS and centrifuged at 800 x g for 10 min. The supernatant was discarded and the pellet was resuspended, washed, centrifuged. The final pellet was resuspended in PBS.

### Example 6

### Hypotonic lysis of red blood cells

For disruption of red blood cells by hypotonic lysis an ammonium chloride solution (BD Lyse™) was diluted 1:10 with water and added at a ratio of 1:16 to whole blood. For lysis of the red blood cells the mixture was incubated for 15 min. in the dark. For separation of cell debris from intact cells the solution was centrifuged for 10 min. at 800 x g. The supernatant was discarded, the pellet was resuspended in PBS, washed again, centrifuged and the pellet was resuspended in PBS.

### Example 7

### Preparation of cells from inner organs of an experimental animal

For the preparation of spleen and thymus cells the respective organ was dissected in a Petri dish and the cells were taken up in PBS. For removal of remaining tissue the cell suspension was filtered through a 100 µm sieve. For obtaining lymphocytes from spleen cells density gradient centrifugation was employed. For thymus cells no further enrichment step was required.

### Example 8

### Depletion of macrophages

Sterile 6-well plates (cell culture grade) were used to deplete macrophages and monocytes through unspecific adhesion. Wells were either coated with KLH (key hole limpet haemocyanine) or with streptavidin and the control peptides. Each well was filled with 3 ml to at maximum 4 ml medium and up to 6x10⁶ peripheral blood mononuclear cells from the immunized rabbit and allowed to bind for 60 to 90 min. at 37 °C in the incubator. Thereafter the lymphocyte containing supernatant was transferred to a centrifugation vial and centrifuged at 800 x g for 10 min. The pellet was resuspended in PBS.

50 % of the cells in the supernatant were used for the panning step; the remaining 50 % of cells were directly subjected to immune fluorescence staining and single cell sorting.

### Example 9

### Depletion of KLH-specific B-cells

Four milliliter of a solution containing keyhole limpet haemocyanine (KLH) was incubated with coating buffer at a concentration of 2 µg/ml in the wells of a multi well plate over night at room temperature. Prior to the depletion step the supernatant was removed and the wells were washed twice with PBS. Afterwards the blood cells were adjusted to a cell density of 2 x 106 cells/ml and 3 ml are added to each well of a multi well plate. Afterwards the multi well plate was incubated for 60 to 90 min. at 37 °C. The supernatant was transferred to a centrifugation vial and the wells are washed twice with PBS and the supernatants are combined in the centrifugation vial. The cells were pelleted by centrifugation at 800 x g for 10 min. and the pellet was resuspended in PBS.

### Example 10

### Enrichment of antigen-specific B-cells

The respective antigen was diluted with coating buffer to a final concentration of 2 µg/ml. 3 ml of this solution were added to the well of a 6-well multi well plate and incubated over night at room temperature. Prior to use the supernatant was removed and the wells were washed twice with PBS. The B-cell solution was adjusted to a concentration of 2 x 10⁶ cells/ml and 3 ml are added to each well of a 6-well multi well plate. The plate was incubated for 60 to 90 min. at 37 °C. The supernatant was removed and the wells were washed two to four times with PBS. For recovery of the antigen-specific B-cells 1 ml of a trypsin/EDTA-solution was added to the wells of the multi well plate and incubated for 10 to 15 min. at 37 °C. The incubation was stopped by addition of medium and the supernatant was transferred to a centrifugation vial. The wells were washed twice with PBS and the supernatants were combined with the other supernatants. The cells were pelleted by centrifugation for 10 min. at 800 x g. The pellet was resuspended in PBS.

### Example 11

### Co-cultivation of B-cells and EL-4 B5 cells

a) The co-cultivation was performed in 96-well multi well plates with round bottom. A basis solution comprising EL-4 B5 cells (1.6 x 10⁶ cells / 15.2 ml) and cytokines in EL-4 B5 medium was prepared. 200 µl of the basis solution was added to each well of the multi well plate. To each well a single B-cell was added by fluorescence activated cell sorting. After the addition of the B-cells the plate was centrifuged for 5 min. at 300 x g. The plate is incubated for seven days at 37 °C.
b) Single sorted B cells were cultured in 96-well plates with 210 µl/well EL-4 B5 medium with Pansorbin Cells (1:20000) (Calbiochem (Merck), Darmstadt, Deutschland), 5 % rabbit thymocyte supernatant and gamma-irradiated EL-4-B5 murine thymoma cells (2 × 10⁴/well) for 7 days at 37 °C in an atmosphere of 5 % CO₂ in the incubator. B cell culture supernatants were removed for screening and the cells harvested immediately for variable region gene cloning or frozen at - 80 °C in 100 µl RLT buffer (Qiagen, Hilden, Germany).

### Example 12

### Cultivation of T-cells

The T-cells were isolated from the thymus of 3-4 week old mice and hamsters, or of 4-5 week old rabbits, respectively. The cells were centrifuged and immediately cultivated or frozen in aliquots of 3 x 10⁷ cells. The thymocytes were seeded with a minimum cell density of 5 x 10⁵ cells/ml of EL-4 B5 medium in 175 cm² culture flasks and incubated for 48 hours at 37 °C.

### Example 13

### Cultivation of macrophages

Macrophages were isolated from the peritoneal cavity of mice and hamsters, respectively, of an age of at least three months. Peritoneal macrophages from mice or hamsters, or blood mononuclear cells from rabbits were cultivated in EL-4 B5 medium at a cell density of at least 1 x 10⁵ cells/ml in 175 cm² culture flasks for 1.5 hours at 37 °C. Afterwards the medium was removed and non-attached cells were removed from the attached macrophages by washing with warm EL-4 B5 medium, followed by cultivation for 48 hours in 35 ml medium.

### Example 14

### Co-cultivation of T-cells and macrophages

T-cells and macrophages were cultivated for 48 hours in separate flasks. Prior to combining both cell populations, the T-cells were centrifuged for 10 min. at 800 x g. The supernatant was discarded and the cell pellet was resuspended in 10 ml medium. The T-cells were adjusted to a minimal cell density of 5 x 10⁵ cells/ml and 10 pg phorbol-12-myristate-13-acetate (PMA) and 5 ng or 50 ng Phytohemagglutinin M (PHA-M) per ml of medium were added. The cultivation medium was removed from macrophages and the T-cell suspension was added to the flasks containing macrophages. After 36 hours of co-cultivation, the cultivation medium was removed and was termed TSN solution. For removal of remaining cells the TSN solution was filtered through a 0.22 µm filter. The TSN solution was frozen at - 80 °C in aliquots of 4 ml.

### Example 15

### Immunofluorescence staining

Depending on the number of cells to be stained the cells were provided in 100 µl medium (less than 10⁶ cells) or 200 µl medium (more than 10⁶ cells), respectively. The fluorescent labeled antibody was diluted with 5 % serum of the experimental animal and FACS buffer to a final volume of 100 µl or 200 µl, respectively. The reaction mixture was incubated on a roller rack for 40 min. at 4 °C in the dark. After the incubation the cells were washed twice at 300 x g for 5 min. The pellet was resuspended in 400 µl PBS and filtered through a 70 µm sieve. The filtered solution was transferred to a FACS-vial and directly before the FACS experiment dead cells were stained by addition of propidium iodide (6.25 µg/ml). If the labeled antibody was labeled with biotin the antibody was detected in a second step with streptavidin labeled Alexa Flour(R) 647 (antibody 197).

### Example 16

### Quantification of IgG

The 96-well multi well plate in which the co-cultivation was performed was centrifuged after seven days of co-cultivation at 300 x g for 5 min. 150 µl supernatant was removed and diluted at a ratio of 2:1 with PBS in a second 96-well multi well plate.

The ELISA was performed as outlined in Example 17.

The antibody was used at a concentration of 50 ng/ml. If the OD was or exceeded 1 after an incubation time of 5 min. a dilution series of from 0.8 to 108 ng/ml IgG was tested.

### Example 17

### Detection of antigen-specific IgG

Antibodies produced by single deposited and co-cultivated B-cells or from B-cells obtained from an immunized experimental animal can be characterized with respect to specific antigen binding. The ELISA was performed at room temperature and the ELISA-solution was incubated between the individual steps on a shaker at 20 x g. In the first step the antigen was bound to the wells of a 96-well multi well plate. If the antigen was a protein it had been diluted in coating buffer and applied directly to the plate. Peptide antigens were bound via the specific binding pair biotin/streptavidin. The wells of the multi well plate can be already coated with soluble CroteinC (CrC) by the manufacturer. If not, the wells were incubated after the immobilization of the antigen with 200 µl blocking buffer. After the incubation with 100 µl antigen solution per well (pre-coated multi well plate) or 200 µl blocking buffer, respectively, non-bound antigen or blocking buffer was removed by washing with wash buffer. The diluted B-cell supernatants were added in a volume of 100 µl per well and incubated. After the incubation the wells were washed. Afterwards the detection antibody was added in a volume of 100 µl per well. The antibody can be either conjugated to horseradish peroxidase or labeled with biotin. The detection antibody was determined with a streptavidin-horseradish peroxidase conjugate. After the incubation the multi well plate was washed and afterwards 50 µl of a substrate solution containing 3,3',5,5' tetramethyl benzidine (TMB) were added per well and incubated for a period as given in Table X. The enzymatic reaction was stopped by the addition of 50 µl sulfuric acid and the optical density was determined at 450 nm and 680 nm with a photometer (Rainbow Thermo ELISA Reader) and the Xread plus-software.

### Example 18

### Isolation of ribonucleic acid (RNA)

The cells from which the RNA had to be isolated were at first pelleted by centrifugation. The cell pellet was lysed by the addition of 100 µl RLT-buffer with 10 µl/ml beta-mercaptoethanol. The cells were resuspended by multiple mixing with a pipette. The solution was transferred to a well of a multi well plate. The plate was shortly shock at 200 x g and frozen at -20 °C.

The isolation of the RNA was performed with the RNeasy® Kit (Qiagen, Hilden, Germany) according to the manufacturer's instructions.

### Example 19

### Reverse transcription polymerase chain reaction

The reverse transcription was carried out in a volume of 20 µl. For each reaction a control was performed with and without reverse transcriptase. Per reaction 1 µl dNTP (each at 10 mM), 0.4 µl oligo(dT)₁₂₋₁₈ (0.2 µg) and 0.6 µl random hexamer (0.03 µg) were pre-mixed and added to 8.5 µl RNA in H2O. The reaction mixture was incubated for 5 min. at 65 °C and directly afterwards transferred to ice. Thereafter 2 µl RT-buffer (10 x), 4 µl MgCl2 (25 mM), 2 µl DTT (0.1 M) and 1 µl RNAse Out™ (40 units) were pre-mixed and added to the ice cold reaction mixture. After an incubation time of 2 min. at room temperature 0.5 µl Superscript™ II reverse transcriptase (25 units) were added. The reaction mixture was incubated for 10 min. at room temperature.

The translation was carried out for 50 min. at 42 °C. After the translation the reverse transcriptase was inactivated by incubation for 15 min. at 70 °C. The cDNA was stored at -20 °C.

### Example 20

### Polymerase chain reaction

The polymerase chain reaction was carried out with the Taq PCR Core Kit (Qiagen, Hilden, Germany) according to the manufacturer's instructions. The PCR was carried out in a volume of 20 µl. The samples were transferred to the Mastercyler® at a temperature of 95°C.

### Example 21

### Sequencing

All sequences were determined by SequiServe (Vaterstetten, Germany).

### Example 22

### Panning on antigen

### a) Coating of plates

Biotin/Streptavidin: Sterile streptavidin-coated 6-well plates (cell culture grade) were incubated with biotinylated antigen at a concentration of 0.5 - 2 µg/ml in PBS at room temperature for one hour. Plates were washed in sterile PBS three times before use.

Covalently bound protein: Cell culture 6-well plates were coated with 2 µg/ml protein in carbonate buffer (0.1 M sodium bicarbonate, 34 mM disodium hydrogen carbonate, pH 9.55) over night at 4 °C. Plates were washed in sterile PBS three times before use.

### b) Panning of B-cells on peptides

6-well tissue culture plates coated with the respective antigen were seeded with up to 6x10⁶ cells per 4 ml medium and allowed to bind for one hour at 37 °C in the incubator. Non-adherent cells were removed by carefully washing the wells 1-2 times with 1x PBS. The remaining sticky cells were detached by trypsin for 10 min. at 37 °C in the incubator and then washed twice in media. The cells were kept on ice until the immune fluorescence staining.

## Claims

1. Use of a synthetic feeder mix for the co-cultivation of B-cells and feeder cells, wherein the synthetic feeder mix is consisting of synthetic components comprising IL-1β, TNFα, IL-10, and
- IL-2 and SAC, or
- IL-6, wherein the feeder mix is for the co-cultivation of rabbit B-cells and feeder cells, or
- IL-6 or IL-2, and SAC, wherein the feeder mix is for the co-cultivation of rabbit B-cells and feeder cells, or
- IL-2 and IL-6, wherein the feeder mix is for the co-cultivation of murine or hamster B-cells and feeder cells, or
- IL-2, IL-6 and SAC, wherein the feeder mix is for the co-cultivation of hamster B-cells and feeder cells.

2. The use according to claim 1, wherein IL-1β, TNFα, IL-2, and IL-10 are recombinant murine IL-1β, murine TNFα, murine IL-2, and murine IL-10.

3. The use according to any one of claims 1 and 2, wherein the synthetic feeder mix comprises IL-6 at a concentration of 10 ng/ml.

4. The use according to any one of claims 1 to 3, wherein the feeder cells are murine EL-4 B5 cells.

## Patentansprüche

1. Verwendung eines synthetischen Füttergemisches zur Co-Kultivierung von B-Zellen und Fütterzellen, wobei das synthetische Füttergemisch aus synthetischen Komponenten besteht, umfassend IL-1β, TNFα, IL-10, und
- IL-2 und SAC oder
- IL-6, wobei das Füttergemisch für die Co-Kultivierung von Kaninchen-B-Zellen und Fütterzellen vorgesehen ist, oder
- IL-6 oder IL-2 und SAC, wobei das Füttergemisch für die Co-Kultivierung von Kaninchen-B-Zellen und Fütterzellen vorgesehen ist, oder
- IL-2 und IL-6, wobei das Füttergemisch für die Co-Kultivierung von Maus- oder Hamster-B-Zellen und Fütterzellen vorgesehen ist, oder
- IL-2, IL-6 und SAC, wobei das Füttergemisch für die Co-Kultivierung von Hamster-B-Zellen und Fütterzellen vorgesehen ist.

2. Verwendung nach Anspruch 1, wobei IL-1β, TNFα, IL-2 und IL-10 rekombinantes Maus-IL-1β, Maus-TNFα, Maus-IL-2 und Maus-IL-10 sind.

3. Verwendung nach einem der Ansprüche 1 und 2, wobei das synthetische Füttergemisch IL-6 in einer Konzentration von 10 ng/ml umfasst.

4. Verwendung nach einem der Ansprüche 1 bis 3, wobei die Fütterzellen Maus-EL-4 B5-Zellen sind.

## Revendications

1. Utilisation d'un mélange nourricier de synthèse pour la co-culture de lymphocytes B et de cellules nourricières, dans laquelle le mélange nourricier de synthèse consiste en des composants de synthèse comprenant l'IL-1β, le TNFα, l'IL-10, et
- l'IL-2 et la SAC, ou
- l'IL-6, dans laquelle le mélange nourricier est destiné à la co-culture de lymphocytes B de lapin et de cellules nourricières, ou
- l'IL-6 ou l'IL-2, et la SAC, dans laquelle le mélange nourricier est destiné à la co-culture de lymphocytes B de lapin et de cellules nourricières, ou
- l'IL-2 et l'IL-6, dans laquelle le mélange nourricier est destiné à la co-culture de lymphocytes B murins ou de hamster et de cellules nourricières, ou
- l'IL-2, l'IL-6 et la SAC, dans laquelle le mélange nourricier est destiné à la co-culture de lymphocytes B de hamster et de cellules nourricières.

2. Utilisation selon la revendication 1, dans laquelle l'IL-1β, le TNFα, l'IL-2 et l'IL-10 sont l'IL-1β murine recombinante, le TNFα murin recombinant, l'IL-2 murine recombinante et l'IL-10 murine recombinante.

3. Utilisation selon l'une quelconque des revendications 1 et 2, dans laquelle le mélange nourricier de synthèse comprend l'IL-6 à une concentration de 10 ng/mL.

4. Utilisation selon l'une quelconque des revendications 1 à 3, dans laquelle les cellules nourricières sont des cellules EL-4 B5 murines.
